# EUROPEAN PATENT APPLICATION

(11) **EP 0 624 383 A1**
(43) Date of publication of application: **17.11.1994**
(21) Application number: 93830198.3
(22) Date of filing: 11.05.1993
(51) Int. Cl.: A61N 1/00

(54) **A neural stimulator**

(71) Applicant: ARIES S.r.l., I-50134 Firenze (IT)
(72) Inventor: Vaiani, Paolo, I-50038 Scarperia (Firenza (IT); Gibelli, Claudio, I-20020 Arese (Milano) (IT); Grassi, Gino, I-50019 Sesto Fiorentino (Firenze) (IT)
(74) Representative: Lanzoni, Luciano

(57) **Abstract**

A neural stimulator comprises two receiving and transmitting devices (15, 1), one located outside the body of a patient, the other implanted in the part of the body to be stimulated and associated with a first set of electrodes (8) through which signals generated by the implanted device (1) are passed into the surrounding tissue; also associated with the implanted device (1) are a second set of electrodes (13, 14) serving to monitor selected bioelectrical functions or activities of the body, and a group of components (10, 9, 2) capable of processing the signals sensed by the monitoring electrodes (13, 14), which therefore can be used as a feedback control in defining at least certain of the parameters of the stimulation signals emitted from the first electrodes (8).

## Description

The present invention concerns a neural stimulator of the type such as can be implanted stably in the human body.

Electrical stimulation is widely used in medicine for the treatment of many diseases; conventional devices, including neural or medullary stimulators used in particular for the treatment of algesis, of various vascular diseases, cases of angina pectoris and other similar conditions, consist in tissue stimulators implanted at least partly within the human body, in a location close to the tissues it is wished to solicit.

The basic principle governing the operation of such stimulators is that of the artificial pacemaker, a system whereby impulses are passed into tissues of the human body, at timed intervals or in response to the value of a control signal, by an electronic generator which can be fitted with suitable means or devices of transducing the signal, for example electrodes or electrode catheters.

By way of example, US Patent 4 800 898 discloses a neural stimulator designed for implantation in a subcutaneous zone, connected with and operating by way of an electrode positioned at various levels of the epidural space afforded by the vertebral column of the patient.

The prior art also embraces apparatus utilizing electrical stimulation for the treatment of various diseases, in which the stimulators comprise means by which to transmit electrical signals, located externally of the body in a suitable position, and means for receiving the transmitted signals located internally of the body in a position close to the tissues it is wished to solicit.

Given the fact that the means for receiving the electrical signals are implanted surgically in the patient, often in particularly delicate areas, and the contingency of the patient having subsequently to undergo treatment with different stimuli at different stages of a cure, the need to remove and replace the receiving means is avoided by designing stimulators capable of changing the type of signal administered to a patient in response to commands, generated externally, corresponding to the changed therapeutical requirements of the patient.

By this expedient, it becomes possible to limit the number of surgical operations on the patient, with obvious benefits.

One example of such an arrangement is described in US 4 459 989. This reference discloses a stimulator for various types of tissue, wherein the stimulus is induced by electrodes implanted in the patient and driven by receiving means, positioned likewise internally of the body, of which the operation is controlled by pulses transmitted from means located outside the body of the patient, the control signal transmitted in this way being variable, by human intervention, in response to changing therapeutical requirements.

With a stimulator of this type, the signal emitted from the electrodes is variable only as a result of human intervention on the transmitting means; the condition of the patient is therefore not monitored by the stimulator, and the change from one type of stimulus to another is made following an analysis effected with means located externally of the body, and in any event means extraneous to the stimulator proper.

An attempt to limit the level of human intervention is discernible in a tissue stimulator as disclosed in US 4 612 934. Implanted or attached to the part of the body it is wished to stimulate, the signal receiving means in this device are associated with sensors capable of monitoring certain physiological parameters and of changing the signals emitted in response to variations in these same parameters.

The signals transduced and returned by the sensors are first converted from analog to digital and then modified by a microprocessor in a manner similar to that by which the stimulator itself controls other functions, namely the timing of the stimulation and the source voltage; in this stimulator, therefore, there is no processing "dedicated" to the monitored physiological signals.

It occurs to the applicant that if an efficient neural stimulation is to be achieved, suitable in particular for use in the treatment of algesis, vascular disease, angina pectoris and the like, the characteristic parameters of the electrical signals should be variable in response to changes in value of the signals evoked in turn by the stimulation. More precisely, by using the electrical signals resulting from variations in potential referred to the action of the cardiac muscle or those resulting from changes in evoked somatosensory potential, it becomes possible to determine algorithms for the parameters dictating the nature of the electrical signal directed to a selected zone of the body, in order to achieve a correct neural stimulation.

A stimulation of this type can be utilized to good effect at least for: peripheral vascular diseases of both the upper and the lower limbs; persistent lumbar pains following a correction of compression or deformation by surgery; spinal arachnoiditis or secondary lumbosacral fibrosis following repeated surgical operations on the rachis or multiple myelographies; phantom limb; painful neuropathies; post traumatic causalgia; post herpetic neuralgia; sympathetic reflex dystrophy; secondary pain spasms from cerebral palsy, multiple sclerosis, dystonia, torticollis, epilepsy; trigeminal neuralgia; angina pectoris; incontinence; deafferentation pain.

The object of the present invention is to provide a stimulator capable of monitoring and memorizing at least two bioelectrical functions or activities, namely Evoked Somatosensory Potential (abbreviated ESP) and electrocardiogram (ECG), and of processing and emitting a stimulation signal correlated to the monitored values of the ESP and ECG signals thus derived. Given that the signals in question are of markedly small amplitude, in particular ESP-derived signals, the necessary analysis is performed on a sample of several stimulation intervals by means of averaging.

The stated object is realized by establishing a two way connection between points located internally and externally of the body of a patient, in such a way that stimulation parameters can be programmed from the outside, and monitored ESP and ECG signals transmitted out from the inside.

The invention allows additionally of varying the stimulation parameters, by means of self-adjusting internal devices, in response to the monitored and processed ESP and ECG signals.

The essential features of the invention include, at least:
- the facility of programming stimulation parameters (frequency, duration, amplitude and polarity of the stimulation signal) from externally of the body of the patient, by way of encoded data transmitted on a radiofrequency, electromagnetic, ultrasound or other conventional carrier;
- monitoring and recording evoked somatosensory potentials, sensed by way of an implanted electrode catheter and averaged using programmable parameters (number of cycles, amplification, pass band), and storing the recorded values, which can then be read from outside the body of the patient by means of a telemetering facility;
- monitoring and recording electrical activity of the heart by way of an implanted electrode catheter and storing the relative signals, the values of which can then be read telemetrically in real time or accessed from memory, particular attention being focused on the S-T deflection of the ECG curve;
- using ESP- and/or ECG-derived signals to determine stimulation parameters automatically, in such a way as to obtain a self-regulating control over the effect and duration of stimulation in response to the effect produced on the evoked signals.

The invention will now be described in detail, by way of example, with the aid of the accompanying drawings, in which:
- fig 1 is a block diagram indicative of a possible embodiment of the neural stimulator according to the present invention;
- fig 2 illustrates a possible configuration of the stimulator based on the block diagram of fig 1;
- fig 3 is a possible flow chart for software used by a stimulator according to the invention.

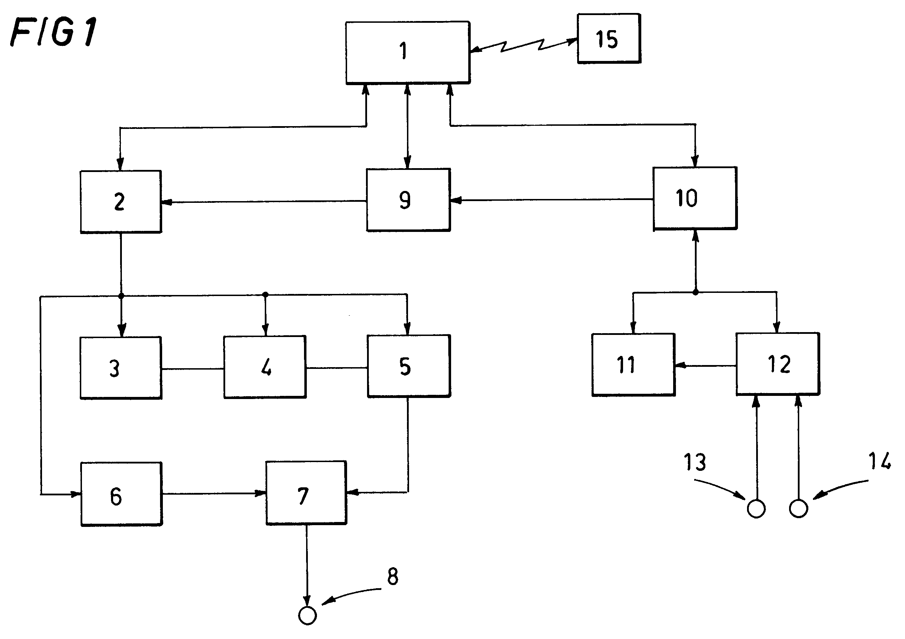

Fig 1 illustrates a functional block diagram of the proposed method of operating a stimulator according to the invention, in the case of an implant.

The blocks denoted 2, 10 and 9 represent three basic programs according to the invention, 2 being a stimulation program, 10 a program for monitoring bioelectrical activities, and 9 a further program allowing interaction between the other two.

All three of the programs 2, 9 and 10 are able to interact via a two way telemetering block 1 with a receiving and transmitting element, indicated by the block denoted 15, located externally of the patient's body; each can thus receive programming data from outside the body as well as transmitting data measured internally of the body, including the current stimulation parameters and the values of memorized bioelectrical signals (ESP or ECG).

The stimulation data will be used by the program 2 to supply dependent circuits with parameters on which to construct a given stimulus; to the end of determining the characteristics of the relative signal, these include dedicated circuits 3, 4 and 5 respectively controlling frequency, duration and amplitude. The signal is emitted ultimately, with frequency, duration and amplitude determined by the relative circuits 3, 4 and 5, and polarity by a further circuit denoted 6, from an output circuit indicated by the block denoted 7.

With this arrangement, signals can be supplied to a set of stimulation electrodes 8 with the desired parameters, on an electrical carrier of which the polarity will be fixed or variable from pulse to pulse.

A monitoring program is transferred to the relative block 10 by way of the telemetering facility 1. This program controls the recording of the evoked signals, and is used to select the reception mode, ESP and/or ECG, according to the type of condition treated.

The blocks denoted 11 and 12 respectively indicate an amplification and a filter circuit controlled by the monitoring program 10, through which signals sensed with two electrodes 13 and 14 are directed; the monitoring program 10 can be used to determine the amplification level and the pass band, also the selection or deselection of the averaging function and the relative parameters, namely the number of periods to be averaged, the repeat value of the averaging cycle and the acquisition time following the trigger.

According to the present invention, the interaction program 9 defines the algorithm determining the manner in which the recorded bioelectrical signals and the stimulation parameters are correlated.

A feedback path is thus established between the information received from the electrodes 13 and 14 and processed by the circuits 11 and 12, and the stimulation signal circuits, effected by way of the interaction program 9 and therefore the stimulation program 2, in such a manner that the stimulation circuits can be piloted to alter the shape of the output signals as dictated by the algorithm entered in the program.

There now follow some examples of the application of a stimulation of this type.

In the case of treatment for angina pectoris, the onset of an ischaemic attack, inducing pain, will be reflected on the ECG by a low takeoff from the QRS complex; the stimulator must respond in such a way as to elevate the takeoff, ceasing stimulation once the correct waveshape has been restored.

In the case of stimulation for vascular diseases, the amplitude of certain premature waves in the ESP signals will vary significantly when the stimulus is effective: accordingly, the action can consist in maintaining the stimulation parameters at values tending to induce such a change in ESP waveshape, thereby obtaining an effective stimulation.

The need may arise in certain instances to measure the potentials evoked by stimulation in positions inaccessible to the implanted electrodes 13 and 14. In such instances, an external ESP recorder can be used, but with the averaging trigger obtained by way of the telemetering facility 1.

From the constructional standpoint, the stimulator (described thus far in purely operational terms) can be embodied from discrete circuit components such as will incorporate solutions of the various blocks mentioned in dedicated hardware.

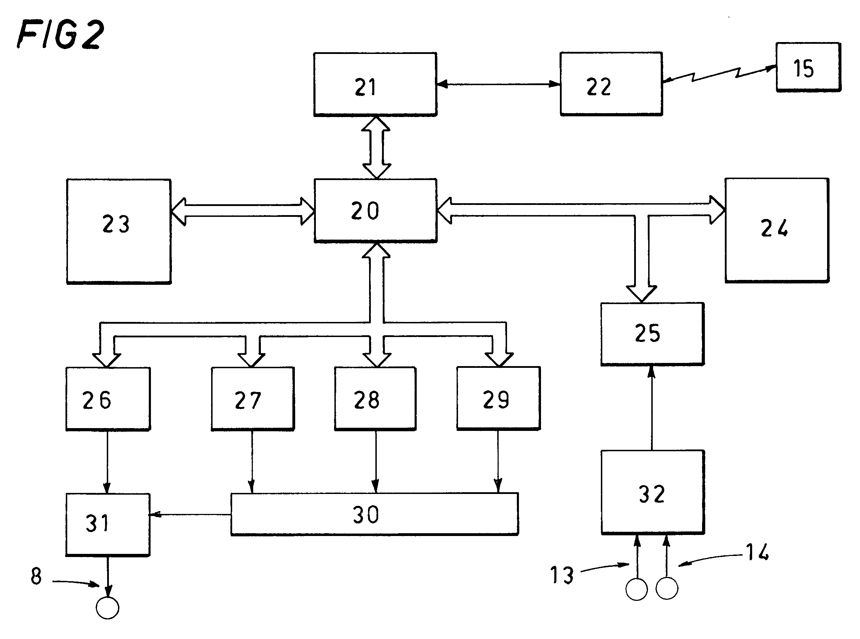

In the example of fig 2, stimulation signals are generated by a microcontroller (or microprocesser) denoted 20; with such a component, the stimulator will be able to perform averaging functions on a numerically broad sample of received signals, as well as piloting the stimulation parameters using feedback control derived from evoked potentials.

Still with reference to fig 2, which illustrates a possible architecture for the system, 22 denotes a telemetering interface allowing the acquisition (via an electrical, electromagnetic or RF coupling) of data and commands from outside the body by way of a coder-decoder or codec 21 compatible with the external receiving and transmitting element 15. 23 denotes an erasable programmable read only memory containing at least one program utilizing commands received telemetrically by way of the interface 22; one such program contained in the EPROM 23 will serve to generate electrical signals, by way of the microcontroller 20, of which the polarity, frequency, duration and amplitude are selectable and determined by dedicated interfaces respectively denoted 26, 27, 28 and 29. The first such interface 26 pilots a selector 31 capable of reversing the polarity of the output signal to the electrodes 8, whilst the remaining interfaces 27, 28 and 29 serve to establish the waveshape and amplitude of the signal at the output of a driver denoted 30.

The EPROM 23 also receives and stores operating instructions for an analog-digital interface 25 in receipt of the signals sensed by the monitoring electrodes 13 and 14 and affording the following functions: the activation of averaging on input signals, selection of the number of values to be sampled, of the interval between one value and the next, and of the type of algorithm to be applied; also the inhibition or enabling of feedback control algorithms which utilize the parameters of evoked potentials, monitored by the electrodes 13 and 14 and amplified and filtered by a circuit denoted 32, to modify the stimulation parameters.

All parameters received telemetrically and creating the set-up configuration for the stimulator remain stored in a random access memory 24, which also holds the data acquired by the A/D interface 25. Accordingly, both the stimulation parameters and the acquired and processed monitoring data can be encoded by the codec 21 and transmitted outwards, away from the body of the patient.

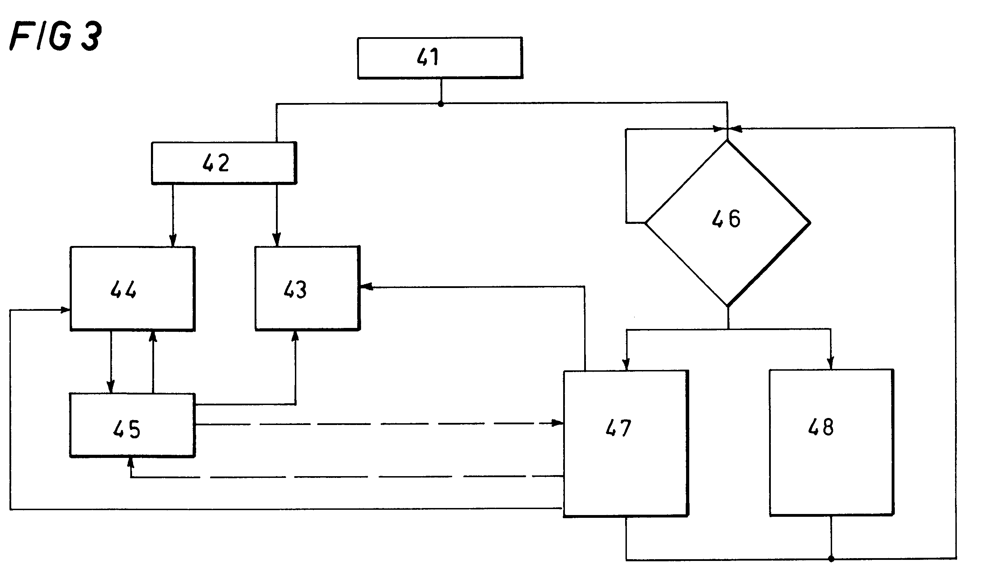

Fig 3 shows a possible flow chart applicable in the case of the software for a solution as in fig 2. 41 denotes an initialization block which will come into operation at power-up, activating the various peripheral functions (timer, trasmit or receive data, etc.) and setting the default parameters for stimulation, for averaging and for evoked potential feedback control if selected, all of which can be altered thereafter by the user.

The blocks denoted 43 and 44 represent processes executed necessarily in real time, activated by a timed interrupt 42 and taking absolute priority: the timer will be set according to the frequency selected for activation of the A/D conversion and averaging processes, both of which are performed by the block denoted 44, and for generation of the stimuli by the block denoted 43. On receipt of an interrupt generated by the timer, the CPU assigns absolute precedence to these two processes.

The data needed for the averaging operation, if in use, is stored in a memory location afforded by the relative block 44 and then relayed to a diagnostics block 45 which will count the algorithms utilized in feedback control of the stimulation parameters. This function will be enabled or otherwise by the user externally.

During the interval between successive interrupts, the CPU will monitor a telemetering input/output interface 46, verifying whether or not there is a current call for data, from a block denoted 48, or a code for a command, from a block denoted 47, to enter or alter stimulation parameters.

In the case of a call for data (evoked potentials, ECG, status of stimulation parameters) the relative block 48 will be enabled concurrently with transfer of the data to the encoding and telemetering system by way of the I/O interface 46.

As regards the entry or alteration of parameters, the new stimulation values (frequency, duration, amplitude, polarity) or the values governing the acquisition of external signals (number of signals to be averaged, type of signal, etc.) will be set by the relative block 47 as and when required.

Where operation in the interactive mode is enabled, the diagnostics block 45 will be programmed with the required type of algorithm and feedback control via the relative block 47 duly activated.

## Claims

1. A neural stimulator, of the type comprising first receiving and transmitting means (15) located externally of the body of a patient, second receiving and transmitting means (1) located internally of the body of the patient substantially in contact with the tissues to be stimulated and affording a first plurality of electrodes (8) by which the tissues are invested with signals generated from the output of the second receiving and transmitting means (1), and means, associated with the second receiving and transmitting means (1), by which to monitor bioelectrical functions or activities,
characterized
- in that means by which to monitor bioelectrical functions or activities consist in a second plurality of electrodes (13, 14); and,
- in that it comprises means (10, 9, 2) located internally of the body of the patient, associated with the second receiving and transmitting means, capable of processing the bioelectrically derived signals sensed by way of the monitoring means (13, 14) and determining at least certain parameters of the stimulation signals emitted from the first plurality of electrodes (8).

2. A neural stimulator as in claim 1, wherein means by which to monitor bioelectrical activities consist in electrodes (13, 14) capable of sensing evoked somatosensory potentials, associated with the second receiving and transmitting means (1) either directly or by way of the first receiving and transmitting means (15).

3. A neural stimulator as in claim 1, wherein means by which to monitor bioelectrical activities consist in electrodes (13, 14) capable of sensing electrocardiographic signals.

4. A neural stimulator as in claim 1, wherein means by which to monitor bioelectrical activities consist in electrodes (13, 14) capable of sensing evoked somatosensory potentials and electrocardiographic signals.

5. A neural stimulator as in claim 1, wherein the electrodes (13, 14) of the monitoring means are connected to the first plurality of electrodes (8) by way of processing means (10, 9, 2) comprising means (11, 12) by which to filter and amplify at least input signals.

6. A neural stimulator as in claim 5, wherein means by which to filter input signals comprise a processing element (11) capable of averaging the values of the signals received.

7. A neural stimulator as in claim 1, wherein signals emitted by the first plurality of electrodes (8) are generated by means comprising:
- a microprocessor (20), connected to the output of a coder-decoder (21) in two-way communication with the first and second receiving and transmitting means (15, 1), the monitoring means (13, 14) and the electrodes (8);
- dedicated circuits (26, 27, 28, 29) piloted by the microprocessor (20) and serving respectively to determine the polarity, frequency, duration and amplitude of the stimulation signals emitted by the electrodes (8);
- at least two memory devices (23, 24) associated with the microprocessor (20), a first capable of storing at least a stimulation program defining the frequency, duration, amplitude and polarity of the stimulation signals emitted by the electrodes (8), and a second capable of storing data supplied by way of the monitoring means (13, 14), in such a manner that stimulation signals can be generated to parameters determined on the basis at least of the stimulation program and of the data supplied by way of the monitoring means (13, 14).

8. A neural stimulator as in claim 7, wherein the monitoring means (13, 14) are associated with the microprocessor (20) by way of an amplification and filter circuit (32) connected to an analog digital interface (25) interposed between the amplification and filter circuit and the microprocessor.

9. A neural stimulator as in claim 7, wherein the first memory device (23) consists in an Erasable Programmable Read Only Memory connected to the externally located first receiving and transmitting means (15) in such a way as to permit of receiving and storing stimulation programs.

10. A neural stimulator as in claim 7, wherein the second memory device (24) is connected to the externally located first receiving and transmitting means (15) in such a way as to permit of receiving and storing default stimulation parameters.
